# EUROPEAN PATENT APPLICATION

(11) **EP 1 946 797 A2**
(43) Date of publication of application: **23.07.2008**
(21) Application number: 07150329.6
(22) Date of filing: 12.12.2002
(51) Int. Cl.: A61N 5/10, G21G 4/08

(54) **Radioactive ion**

(30) Priority: 12.12.2001 US 339313 P
(62) Divisional of application: 02787004.7
(71) Applicant: The Univ. of Alberta, The Univ. of British Columbia, Carleton Univ., Simon Fraser Univ. and The Univ. of Victoria, Vancouver, British Columbia V6T 2A3 (CA)
(72) Inventor: Vincent, John S., Vancouver British Columbia V5Y 2PB (CA); Ruth, Thomas J., Vancouver British Columbia V6S 1M4 (CA); Zyuzin, Alexander, Vancouver British Columbia V6T 1X7 (CA); D'Auria, John Michael, North Vancouver British Columbia V7G 1B8 (CA); Ottewell, David F., Vancouver British Columbia V4M 4C7 (CA); Harshman, Dale R., Lyden, WA 98264 (US)
(74) Representative: Andersson, Inga-Lill

(57) **Abstract**

The present invention relates to a method for implantation of Xe isotopes in a matrix for production of ¹²⁵I sources that do not shed radioactive atoms. ¹²⁵Xe implanted at 12kV in steel, titanium and gold does not evol ve after more than 10 half-lives (380h) and ¹²⁵I from the decay of implanted ¹²⁵Xe is equally stable for 2 half-lives (120d). The matrix having radioxenon implanted is useful as a medical device, for instance as a "seed" for radiotherapeutic uses or in production of stents. Methods of treatment utilizing such devices are also encompassed by the present invention.

## Description

### FIELD OF THE INVENTION

The present invention relates to a method for producing matrices having radioxenon embedded in them. In one embodiment of the invention, the radioxenon decays to ¹²⁵I and thus the matrices so produced are useful as sources of gamma radioactivity, or photon and electron emission radioactivity, especially for radiotherapeutic and imaging uses. In another embodiment of the invention ¹²⁷Xe is implanted and the emissions from that isotope are used for imaging applications. The invention also relates to radioactive matrices produced by the method and to therapeutic, diagnostic and imaging methods using the radioactive matrices.

### DESCRIPTION OF THE BACKGROUND ART

¹²⁵I encapsulated in metallic "seeds" has been used for many years for treatment of prostate cancer. Also ¹²⁵I has been incorporated into various metallic plaques for conformal treatment of other tumours, especially melanoma in the eye, and metal bridges, called stents, to prevent re-occlusion of arteries opened by balloon angioplasty. A general discussion of brachytherapy devices can be found in Principle and Practice of Brachytherapy ed. Subir Nag, MD, Futura (1997). All of these applications utilize photons emitted from ¹²⁵I, which does not emit beta particles. Historically, the ¹²⁵I was chemically bound to materials called ion exchange resins which were encapsulated in non-contaminated materials to prevent release into body fluids during the course of the treatment. US Patent 6,060,036 to Armini discloses a method of implanting a pure precursor isotope, viz. ¹²⁴Xe, into the device which is subsequently made radioactive by neutron activation. The central feature of Armini's process is mass spectroscopic separation of natural precursor material, which results in savings relative to the cost of previously separated isotopically pure material. Subsequent to activation, the device is further encapsulated in an outer casing to complete the manufacturing process.

There are at least three disadvantages of the seed manufacturing process of Armini:
(1) The process has three steps, implantation of the precursor isotope, neutron activation and encapsulation of the activated device. In order to arrive at the desired source strength and therefore target dose, the overall quality control is the convolution of the three processes each of which has its own inaccuracies. It is more difficult to achieve consistent results than if there were to be a single step.
(2) Though several substrates can by used in Armini's method, viz. aluminum, titanium, silicon, silicon dioxide, alumina, copper or rhodium, the substrates are also activated along with their natural contaminants by neutrons in the irradiation step. Expensive, high purity substrate materials can be used to control this unwanted radioactivity, but even in this case, decay time and quality control techniques must be allocated to assure that this radioactivity does not arrive at the patient. Depending on the character of the unwanted radioactivity, there may be additional dose both in magnitude and spatial distribution to the patient beyond that expected from a medical treatment plan based on the primary activity.
(3) Because relatively high precursor isotope beam currents( 10-20microamperes), substrate sputtering will erode the surface which may result in loose surface radioactivity on activation and making the additional device encapsulation a requirement.

Radioactive ion implanted brachytherapy sources have been under development in industry¹ and a Canadian government laboratory² for a few years. In the most common application, beta emitting ³²P implanted stents is used in conjunction with percutaneous transluminal coronary angioplasty ("balloon angioplasty", PCTA). ³²P has been shown to be effective at inhibiting restenosis. The use of ¹²⁵I for various brachytherapy applications, notably jacketed seeds, is also well known.

### SUMMARY OF THE INVENTION

¹²⁵I emits copious amounts of photons in the range of 27 to 35KeV. The present invention provides a method for producing matrices having useful amounts of ¹²⁵I deposited at a shallow depth within the matrix. The present invention also provides a method for implanting ¹²⁷Xe in a matrix.

The present invention is a single step technique whereby a radioisotope or a precursor radioisotope is produced, isotopically mass separated and implanted beneath he surface of a substrate where it decays. The decay produces either a desired emission or a desired therapeutic daughter. Since the substrate is never activated, the widest possible variety of materials may be used, including all those excluded by Armini. For medical uses, the material used for the matrix is limited only by biological compatibility. Since the only deposited isotopes are mass separated and accelerated to the desired energy (voltage), with currents less than 0. 1 microampere there will be negligible sputtering. Consequently, negligible radioactivity will be exposed on the surface of the substrate to be released to body fluids and to deliver an unacceptable radiation dose outside the treatment region. A consequence of this fact is that these substrates may be used directly without further encapsulation, subject only to limitations related to stability of the substrate in the application environment (e.g. body fluids for medical uses). In the case that x-ray markers are required, the substrate may comprise heavy element marker materials. Again, in medic al uses, these markers should be biologically compatible.

Use of ¹²⁵Xe-(17h) precursor plays an important role in the present invention. Because xenon is a noble gas it can be extracted quantitatively (100%) by simple heating alone from certain materials used to produce it. Being a noble gas, xenon does not stick to clean metal surfaces. Furthermore, transportation of this gas from its point of production, an accelerator target or a fission target in the core of a reactor, is also quantitative and rapid by vacuum pumping, with or without trace quantity of helium. This minimizes difficulties in handling of radioactive material. Furthermore, ionization of noble gases by the technique of electron cyclotron resonance (ECR) is the most efficient method of deriving the required radioxenon beam, also because of its chemical inertness. Taken altogether, the properties of ¹²⁵Xe make it a desirable precursor for the efficient production of ¹²⁵I activated devices in a single step process.

¹²⁷Xe has several gamma emissions, the main of which are at energies of 172, 202 and 374 keV. Thus, the present invention can also be applied using ¹²⁷Xe for implantation to image devices otherwise used for therapeutic purposes. For example, it is known that certain prostate seeds will migrate from the implantation site in the patient and this can be hazardous if they find their way into the circulatory system, forming occlusions and the like. One could use ¹²⁷Xe implanted seeds as mechanically exact duplicates ¹²⁵I implanted seeds (or for that matter ¹⁰³Pd seeds) to measure their mobility using nuclear medicine techniques. While one can x-ray for seed position changes if a heavy metal marker is part of the construction, ¹²⁷Xe implanted seeds can be "coded" easily with different activity levels and distributions to remove ambiguities. Also it is quite easy to implant a small admixture of ¹²⁷Xe into a ¹²⁵I seed to make the seed self-imaging.

In this embodiment of the invention, it is emission from the implanted. ¹²⁷Xe that is utilized in the imaging technique and decay to the daughter iodine is irrelevant. All of the advantages enjoyed due to use of ¹²⁵Xe are also enjoyed by use of ¹²⁷Xe, except of course those due to decay to a useful daughter isotope.

### BRIEF DESCRIPTION OF THE DRAWINGS

Figure 1 shows the range and straggling of ¹²⁵Xe implanted in iron is shown as a function of implantation energy.
Figure 2 shows the longitudinal (Figure 2A) and transverse (Figure 2B) distribution for 500 trajectories from an incident, point beam. Pictures of the paths of typical 12kV ¹²⁵Xe ions in iron were generated using the monte-carlo program TRIM³.
Figure 3 shows the charge state distribution of stable ¹³⁶Xe generated in the ECR in Example 1.
Figure 4 is a schematic of an apparatus useful for radioxenon implantation according to the invention. In Figure 4, the various numbered items are:
   1. TISOL, Test Isotope Separator On-line used for quantitative measurements of stable and radioxenon implantation;
   2. 100 ton beam dump for 500MeV proton beam;
   3. Carbon beam stop for 500 MeV proton beam;
   4. 50gram/cm² cesium metal target for the production of radioxenon with 500 MeV proton beam;
   5. Radioxenon gasline from production target to ion implanter;
   6. Electron cyclotron resonance ion source for ionizing radioxenon;
   7. Quadrupole focus magnet #1 of ion implanter;
   8. Sextupole magnet of ion implanter for second order focal corrections;
   9. Octupole magnet of ion implanter for high order focal corrections;
   10. Dipole magnet #1 of ion implanter, for isotope mass dispersion;
   11. Collector of unused radioisotopes, generally with charge states greater than one;
   12. Dipole magnet #2 of ion implanter, for isotope mass dispersion;
   13. Quadrupole focus magnet #2 of ion implanter;
   14. Quadrupole focus magnets #3,4,5 of ion implanter specific for ¹²⁵Xe team.
Figure 5 shows several histories of ¹²⁵Xe (148, 243keV lines) in Fe (Figure 5A), Ti (Figure 5B) and Au (Figure 5C) for implantation voltages of 12 and 22kV and ¹²⁷Xe (172,202,374keV lines) in Fe sources (Figure 5D) implanted at 12kV in stainless steel foil over periods 157h and 652h respectively.

### DETAILED DESCRIPTION OF THE INVENTION

¹²⁵Xe and ¹²⁷Xe production:
¹²⁵Xe can be produced in several kinds of nuclear reactions. Two reactions are of interest for efficient recovery. The first reaction is cesium metal spallation by high-energy protons and the second is fission of ²³⁵U in a reactor. Both reactions are capable of supplying ¹²⁵Xe at the rate of a few curies per hour, which is suitable for commercial production of ¹²⁵I implanted devices.

The spallation process of 500 MeV protons on molten cesium metal is described in Journal of Radioanalytical Chemistry, Vol. 65, No. 1-2, pp.17-29, (1981) by IS. Vincent et.al. Based on ¹²⁵Xe cross sections measured there a 50g/cm² target of cesium would yield 0.7 Ci/hr with a 10 microampere proton beam. Nearly 100% of this activity is available for implantation.

¹²⁵Xe and ¹²⁷Xe along with other isotopes of noble gases, including both xenon and krypton, are commonly produced by fission of ²³⁵U in a nuclear reactor. In order to extract these gases efficiently, the target material, uranium is deposited from solution onto a carbon-felt material to achieve the maximum surface area. The assembly must then be encapsulated in a container, which is compatible with the irradiation facility. Irradiation to fluence of 10¹⁷ neutrons will provide about several hundred millicuries of ¹²⁵Xe per gram of target uranium. Similar amoungs of ¹²⁷Xe would be produced. It is estimated that at least 10% of this activity could be extracted for implantation.

### General arrangement and implantation apparatus:

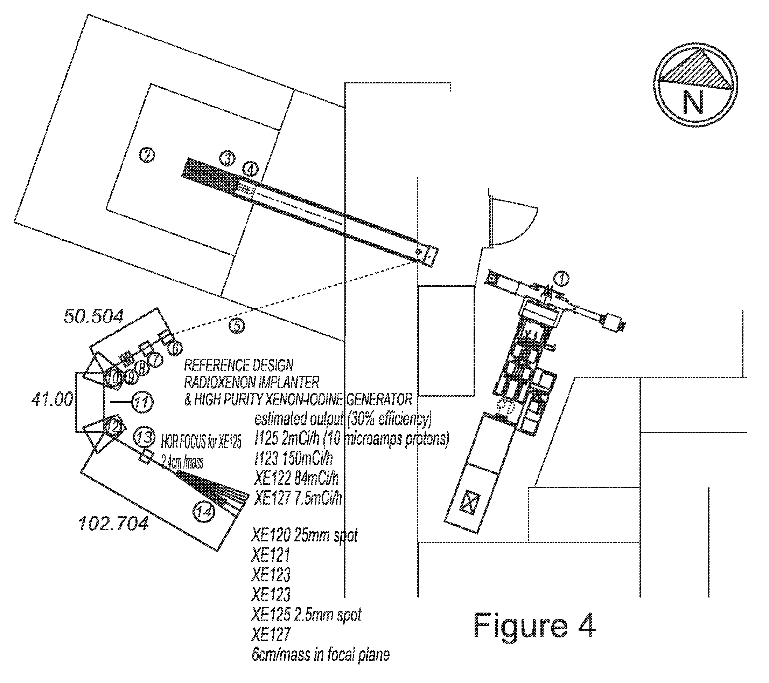

The general arrangement of the target system and ion implanter for accelerator production is shown in schematic plan view in Figure 4. This design allows for continuous production and implantation. The cesium target is located 6 meters below grade in a 100 ton proton beam dump. The dotted line marked radioxenon gas line consists of a 5mm diameter metal tube connecting the target and an ECRIS (electron cyclotron resonance ion source) at grade level. This source is of a type described in Nuclear Instruments and Methods in Physics Research B62, pp. 521-528 (1992) by L. Buchmann, et.al. The mass separator consists of two dipole magnets, two quadrupole magnets, one sextupole and one octupole magnet arranged to provide sufficient dispersion (6 cm per mass unit) and second order correction to provide a 2.5 mm focus of the ¹²⁵Xe beam.

The arrangement for reactor production would use the same reference design radioxenon implanter with the ECRIS adapted to accept the ²³⁵U target in a batch production mode from the reactor.

Prior art in the making of brachytherapy sources is described in the patent of Armini in some detail and more generally in the text of Nag, referenced above. The principal improvement of our reference design is the second order correction magnets, not generally available on commercial implanters. This second order correction achieves raw xenon beam diameters of
2.5 mm and can be reduced to less than 1 mm diameter by collimation and corresponding loss in beam strength. The advantage of small ¹²⁵Xe beams is that they may be used to implant specified non-uniform radioactivity profiles in special medical devices such as stents or eye plaques by articulating the substrate or the beam. Specified non-uniform radioactivity profiles are useful in stents and plaques to shape the radiation dose profile to fit a tumour or to spare blood vessel walls at the ends of a stent. This feature is not useful in seeds for prostate treatment, where the spatial configuration of many seeds is used to shape the dose at the tumour site.

The implantation apparatus might be improved to provide better quality control. Such improvements relate to the rate of deposition and its spatial distribution as outlined above. These improvements require precise monitoring of the rate of ionization of the ¹²⁵Xe. This can be accomplished by continuous monitoring of the ¹²⁵Xe inventory by monitoring of its gamma emissions using a germanium counter and by regulating the rate that the inventory is delivered to the ion source. ECRIS ion sources are known to operate with good stability for days if the gas pressure is well regulated. Pressure stability of 1-2% is possible using fixed noble gas leaks. The corresponding beam stability will be a fraction of this value.

As discussed above, wider varieties of device substrates are possible compared to Armini because the substrates are not activated by beams that can cause nuclear reactions. There are no limitations on the kind of materials that can be used, except those imposed by the environment of the application of the device. For example, use in medical devices will likely require biological compatibility. Devices may or may not be encapsulated depending on this limitation. For non-encapsulated medical devices, it may be desirable to use heavy metal substrates such as gold or platinum that are designed as x-ray markers. This avoids the necessity to fabricate composite devices where the marker and substrate are different materials.

The method of preparing devices for implantation has been generally described in the art, e.g by Armini for prostate therapy seeds in U.S. Patent 6,060,036, and these techniques can be applied in the present invention. Matrices implanted according to the present invention can be further encapsulated, e.g. by laser beam welding. For non-encapsulated seeds the material will be machined to the final size and shape, usually 4.5mm long by 0.8mm diameter in the case of a radiotherapy seed. Of course the surface may or may not be plated with substrate material, preferably up to 1-micron thickness, for example by electron beam sputtering and this surface film may contain all of the implanted radioactivity as determined by the implanter beam energy. Stents of stainless steel are available commercially from several suppliers such as the popular NIR stent from Medinol. Ltd. Tel Aviv, Israel. Eye plaques can be fabricated locally by numerically controlled machine techniques. They may consist of biologically compatible material which is shaped to fit the eye and may be sputter plated with metal coatings of compatible material up to several microns thickness for ¹²⁵Xe implantation in either uniform or specified areal distribution.

Radioactivity levels required for therapeutic plaques and seeds are of the order of one millicurie or less ¹²⁵I. The ratio of ¹²⁵Xe to ¹²⁵I activity is 84.2:1 (a device requiring 1 millicure of ¹²⁵I will require 84.2 millicuries of implanted ¹²⁵Xe). Stents require only 10-50 microcuries of ¹²⁵I to be effective.

In general, four factors are important to determining the feasibility of using ¹²⁵Xe or ¹²⁷Xe implantation according to the present invention: 1) production rates of ¹²⁵Xe and ¹²⁷Xe; 2) implant system efficiency; 3) stability of the implanted species against losses and, in medical uses 4) radiobiological effectiveness of the application. Methods for efficient production of ¹²⁵Xe are described above and are known in the prior art. Also, the efficacy of radiotherapy in various medical uses is considered known in the art; the various modes of administration and doses required for different intended therapeutic, imaging and diagnostic uses are considered well-known or within the skill of the medical practitioner to develop.

The present invention provides a method for efficiently implanting ¹²¹Xe in a matrix to provide stably implanted ¹²⁵Xe, with the result that, after sufficient decay, the matrix will contain an amount of ¹²⁵I stably implanted in the matrix. The method also provides for efficient implantation of ¹²⁷Xe.

Thus the present invention can be a method for producing a matrix implanted with ¹²⁵I comprising:
i) implanting ¹²⁵Xe in the matrix;
ii) allowing decay of the radioisotope of ¹²⁵Xe to ¹²⁵I; and
iii) isolating the portion of the matrix containing the ¹²⁵I.
   The matrix can be a metal, such as titanium or copper. Alternatively, the matrix can be a non-metallic, biocompatible material, such as silicon or a plastic such as teflon. If necessary, the matrix can be covered with a biocompatible material after implantation.

The radioisotope of Xe used in the invention can be either ¹²⁵Xe or ¹²⁷Xe. ¹²⁷Xe is useful for imaging applications utilizing gamma emission. ¹²⁵Xe is useful in applications where ¹²⁵I is the desired implanted isotope. The method of the invention preferably yields a matrix in which the Xe is implanted with a depth distribution such that 99% or more of the Xe is from 0.1 to 100 angstroms, preferably one in which 99% or more of the Xe is from 0.1 to 50 angstroms, from the surface of the matrix. In a preferred embodiment, the mean depth of Xe atom distribution is from 40 to 50 angstroms from the surface of the matrix. For ¹²⁷Xe implantation, implantation can be to a depth of up to 1 mm, as the emitted photons are of sufficient energy to escape the matrix even at that depth.

Figures 5A-5D show the rate of conversion of Xe radioisotopes to ¹²⁵I. In a preferred embodiment of the invention the radioisotope of Xe is ¹²⁵Xe and the decay is allowed to proceed for from 2 to 150 hours. In another embodiment, the radioisotope of Xe is ¹²⁷Xe and emission from the ¹²⁷Xe is utilized in the intended application. The invention also encompasses medical devices comprising a matrix prepared according to the method of the invention. Medical devices such as a stent or a radiotherapy seed are common applications of the method of the invention.

The present method is distinguished from the prior art, e.g. as represented by U.S. Patent 6,060,036, in that ¹²⁵Xe is used directly for implantation rather than being produced in situ in the matrix by neutron bombardment of the matrix after implantation of ¹²⁴Xe. Thus, the invention also encompasses medical devices that comprise ¹²⁵I, ¹²⁵Xe and a matrix, wherein the ¹²⁵Xe is obtained by a method other than neutron capture by ¹²⁴Xe. Furthermore, because there is no need for irradiation of the matrix after implantation, the invention also encompasses matrices, and devices made from them, that comprise more than 0.001% by weight of at least one element selected from the group consisting of iron, cobalt, zinc, manganese, platinum and iridium.

The invention being thus described, the invention will be further understood in view of the following examples, which illustrate, but do not limit, the invention. The scope of the invention is limited only by the claims following.

### EXAMPLE 1 - Radioxenon implantation into metal foils

The rationale of use of the radioxenon precursor is that it is readily extracted from production targets and efficiently ionized in plasma ion sources. This Example describes two experiments. The first experiment establishes the stability of ¹²⁵Xe(16.9±0.2h), its daughter ¹²⁵I (59.40±0.01d) and ¹²⁷Xe(36.4±0.1d) in metal foils. The second experiment measures the practical system transmission for delivery of singly ionized radioxenon to target devices.

### Materials and methods:

The apparatus used for these experiments is called TISOL⁴ (Test Isotope Separator On-Line) at TRIUMF. It consists of a target box in the accelerator proton beam, an adj acent ionizer, a magnetic spectrometer and various beam-focusing elements. The mass resolution M/ΔM=250 is low but sufficient for this work. The target-ion source combination can be electrically biased (up to 20kV) to provide acceleration potential for reaction products. The beam path is about 10 m and whole system operates at 3 x10⁻⁶ torr to minimize charge exchange of the beam in flight. For the on-line studies, a beam of 0.7µA, 500 MeV protons was focused on 5 grams of lanthanum carbide which was electrically heated up.to 1200°C for the evolution of radioxenon.

The ECR (electron cyclotron resonance) ion source⁵ was driven by 300W, 6 GHz rf power and radioxenon from the adjacent target was drifted into its quartz plasma chamber.

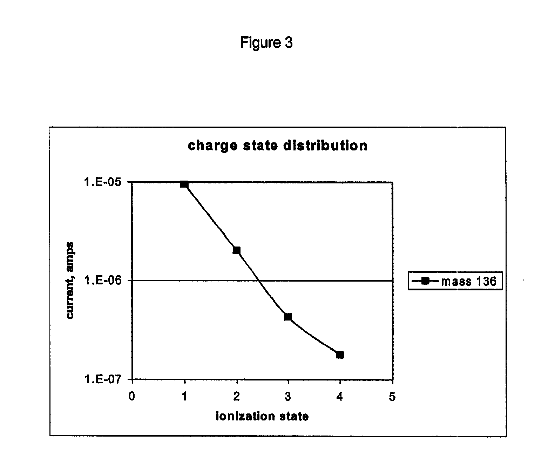

Electrons are confined both longitudinally and radially by magnetic fields in this ionizer so that there are multiple chances for the xenon to be ionized. An undesirable artifact of this process is the emission of multiple charge states as illustrated in Figure 3 for stable xenon mass 136. The other xenon isotopes behave in a similar fashion. The stable mass 136 isotope was used for assessing the charge distribution as it provides more accurate measurement of the charge state distribution. Also, use of a stable isotope for this experiment removes the need for unnecessary handling of radioactive material. It is not possible to substantially change the distribution of charge states with this source configuration. Optimization for the experiments consists of maximizing xenon output through adjustment of the confinement field, rf excitation, extraction field and external ion optics.

A 12kV beam from the ion source was magnetically separated, focussed on mass defining slits and transmitted to an implantation chamber. Target metal foils of titanium, steel and gold were electrically isolated from ground so that additional voltage could be applied to vary the penetration depth. Clean mass separation of radioxenon masses 122 to 127 was accomplished by observing gamma ray spectra at the implantation foil using an intrinsic germanium detector. Only mass 125 and 127 were used for implantation. ¹²⁵Xe (17h) and ¹²⁷Xe (36.4d) were the species of interest for the evaluation of stability at small implantation depths but ^{125m}Xe (57s) and ^{127m}Xe(69s) were of great utility in adjusting the parameters of the mass separator for optimal transmission.

Target foils were counted using another intrinsic germanium detector for characteristic gamma rays of the radioxenons and an intrinsic silicon detector was used to count the 35 kV gamma from the ¹²⁵I daughter. Both detectors were calibrated with standard sources to establish the counting efficiency. In order to check the stability of the ¹²⁵I daughter the foils were soaked in normal saline at room temperature and 55°C for 3 days. Afterward, the solutions were taken to dryness at room temperature and the residue counted for free ¹²⁵I.

For measurement of the system transmission, 5mCi of pure ¹²⁷Xe, produced elsewhere, was mixed with 1 atmosphere of natural xenon in a 0.5 liter container and connected to a vacuum flask of known geometry through a fixed gas leak of about 10⁻⁶ atmosphere milliliters per second. The leak rate was then calibrated by counting the activity accrued after one hour. Two such measurements were made to account for decay and pressure loss during the experiments. Subsequent to calibration, the xenon leak containing stable masses 124,126,128,129,130,131,132,134, 136 together with radioactive mass 127 was connected to the ECR-TISOL system and metal foils at the mass defining slits were implanted with ¹²⁷Xe. Stable xenon masses were detected by an electrometer connected to the metal foils and were used to set the system parameters for ¹²⁷Xe.

### Foil implantation results:

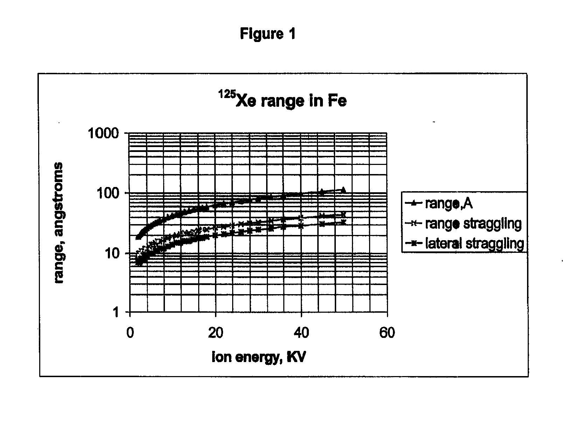

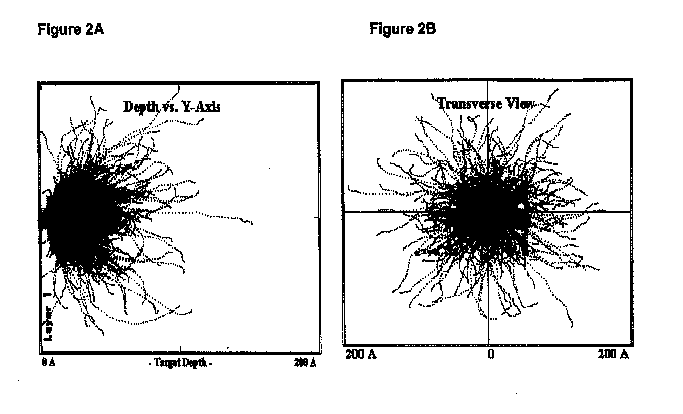

As can be seen in Figure 1, ¹²⁵Xe deposited at 12kV has a mean range of about 47 Å (0.047µ). The mean range and lateral straggling is 21 and 15 Å respectively as shown in Figures 2A and 2B.

Figures 5A-5D show of several histories of ¹²⁵Xe (148, 243keV lines) in Fe, Ti and Au for implantation voltages of 12 and 22kV and ¹²⁷Xe (172,202,374keV lines) in Fe sources implanted at 12kV in stainless steel foil over periods 157h and 652h respectively.

An estimate of radioactivity loss over these periods can be calculated by fitting the decay curves to a simple exponential and comparing to known half-lives. A more sensitive test is the measurement of residual ¹²⁵I and a comparison with the calculated ¹²⁵Xe at end of bombardment taken from the fitted data.

¹²⁵I left in the ¹²⁵Xe implanted foil above was determined by counting the 35.5keV(6.6%) gamma using an intrinsic silicon detector. In the table below the expected quantity of implanted ¹²⁵I is calculated for the radioxenon activities measured above. The subscript "c" denotes calculated ¹²⁵I activity based on fits to the radioxenon data and "o" is the observed ¹²⁵_{I.}

| **HT** | **mat'I** | **Range, Å** | **strag, Å** | **¹²⁵Xe@EOB Bq ± Bq** | | **decay, hr** | **¹²⁵I Bq_{c}** | **¹²⁵I ±Bq_{c}** | **¹²⁵I Bqₒ** | **¹²⁵I ±Bqₒ** | **Ratio** | **±** |
|---|---|---|---|---|---|---|---|---|---|---|---|---|
| 12kv | Fe | 47 | 21 | 113383 | 979 | 209 | 1211 | 120 | 1181 | 48 | 0.98 | 0.11 |
| 22kv | | 67 | 28 | 91545 | 823 | 244 | 961 | 97 | 965 | 49 | 1.00 | 0.11 |
| 12kv | Ti | 79 | 32 | 152308 | 1096 | 257 | 1589 | 158 | 1442 | 41 | 0.91 | 0.10 |
| 22kv | | 113 | 43 | 126675 | 1012 | 946 | 946 | 99 | 1003 | 79 | 1.06 | 0.13 |
| 12kv | Au | 33 | 27 | 90384 | 835 | 945 | 676 | 69 | 595 | 48 | 0.88 | 0.13 |
| 22kv | | 47 | 37 | 90410 | 837 | 293 | 927 | 94 | 931 | 40 | 1.00 | 0.11 |

Leaching tests for 3 days in room temperature and 3 days in 55°C normal saline revealed no ¹²⁵I activity in the leachant for 12kV implanted Fe, Ti and Au foils at the level of 3 Bq.

### Implantation efficiency:

Two measurements of the ¹²⁷Xe leak rate were made by connecting the 0.5 liter glass flask to a calibrated 1 liter well container. The average rate for two measurements was 19.8±1.9kBq/hr. This source was connected to ECR- TISOL for one hour to implant ¹²⁷Xe in aluminum with 12kV accelerating voltage. The yield was 4.6±0.3kBq of implanted ¹²⁷Xe accelerated in the one-plus charge state. The system efficiency for this experiment was thus 23±3% for the single charge state.

### Discussion:

The retention of ¹²⁷Xe, ¹²⁵Xe and its daughter ¹²⁵I in metal surfaces at depths of 33 to 113 Å has been shown to be quantitative at the level of 10% uncertainty. The experiment utilizing ¹²⁷Xe shows the time of retention of Xe for 30 times the half-life of ¹²⁵Xe. The efficiency of radioxenon single charge state ionization by an ECR ion source of the type described in ref. 5 was found to approach 23%. These results suggest that it would be reasonable to proceed with design of an ion implanter for radioxenon. In order to fabricate biologically effective medical devices using this scheme, the lanthanum carbide target would be unsatisfactory because of the extreme energy dependence of evolution of radioxenon from the target.

In previous work⁶ the production radioxenon at TRIUMF from proton bombardment of metallic cesium has indicated a production cross section of 48± 4mb at 482 MeV and it is shown to increase slightly for energies down to 200MeV. Extraction of the xenon from this target was known to be quantitative within 10%. Thus 10µA on a typical 50g/cm² target would be capable of delivering the equivalent of 8.5mCi/h ¹²⁵I as the xenon parent. The 23% transmission in the one-plus state as found here provides for implantion at the rate of 2 mCi/h with the 10 microampere beam.

The present specification cites various articles of the scientific and patent literature. Each such article is hereby incorporated by reference in its entirety and for all purposes by such citation.

### References:

1. Implant Sciences Corporation, 107 Audubon Road #5, Wakefield, MA 01880-1246. www.implantsciences.com/
2.Forschungszentrum Karlsruhe, GmbH, www.fzk.de/
*3.* The Stopping and Range of Ions in Matter, James F. Ziegler, IBM Research 28-0, Yorktown, NY, Ziegler@Watson.IBM.Com
4.J.M. D'Auria, L. Buchmann, M.Domsky, P.McNeeely, G.Roy, H.Sprengei, and J. Vincent, NIM, B70, (1992)75-79.
5. L.Buchmann, J.Vincent, H. Sprenger, M.Domsky, J.M.D'Auria, P.McNeely, and G. Roy, NIM B62(1992)521-528.
6. J.S.Vincent, A.H. Dougan, D.M. Lyster and J.W.Blue, J. Radioanal. Chem. 65,(1981)17-29

1. A method for producing a matrix implanted with ¹²⁵I comprising:
   i) implanting ¹²⁵Xe in the matrix;
   ii) allowing decay of the ¹²⁵Xe to ¹²⁵I; and
   iii) isolating the portion of the matrix containing the ¹²⁵I.
2. The method of claim 1, wherein the matrix is a metal.
3. The method of claim 2, wherein the metal is titanium or copper.
4. The method of claim 1, wherein the matrix is a non-metallic, biocompatible material.
5. The method of claim 1, further comprising covering the matrix with a bioc:ompatible material.
6. The method of claim 1, wherein the ¹²⁵Xe is implanted with a depth distribution such that 99% or more of the ¹²⁵Xe is from 0.1 to 100 angstroms from the surface of the matrix.
7. The method of claim 1, wherein the mean depth of ¹²⁵Xe atom distribution is from 40 to 50 angstroms from the surface of the matrix.
8. The method of claim 6, wherein the mean depth of ¹²⁵Xe atom distribution is from 40 to 50 angstroms from the surface of the matrix.
9. The method of claim 1, wherein the decay is allowed to proceed for from 2 to 150 hours.
10. A medical device comprising a matrix prepared according to the method of claim 1.
11. A medical device comprising a matrix prepared according to the method of claim 2.
12. The medical device of claim 10 that is a stent.
13. The medical device of claim 10 that is a radiotherapy seed.
14. A medical device that comprises ¹²⁵I, ¹²⁵Xe and a matrix, wherein the ¹²⁵Xe is obtained by a method other than neutron capture by ¹²⁴Xe.
15. The medical device of claim 14, that comprises more than 0.001% by weight of at least one element selected from the group consisting of iron, cobalt, zinc, manganese, platinum and iridium.
16. The medical device of claim 14 that is a stent.
17. The medical device of claim 14 that is a radiotherapy seed,
18. A matrix comprising ¹²⁷Xe implanted with a depth distribution such that 99% or more of the ¹²⁷Xe is from 0.1 to 10⁷ angstroms from the surface of the matrix.
19. The matrix of claim 18 that is a biocompatible material.
20. A medical device comprising a matrix implanted with ¹²⁷Xe.

## Claims

1. A matrix comprising ¹²⁵Xe implanted with a depth distribution such that 99% or more of the ¹²⁵Xe is from 0.1 to 100 angstroms from the surface of the matrix.

2. A matrix according to claim 1, comprising ¹²⁵Xe implanted with a depth distribution such that 99% or more of the ¹²⁵Xe is from 0.1 to 50 angstroms from the surface of the matrix.

3. A matrix according to claim 1 or 2, wherein the mean depth of ¹²⁵Xe atom distribution is from 40 to 50 angstroms from the surface of the matrix.

4. A matrix according to any one of the claims 1-3, wherein the ¹²⁵Xe has decayed to an amount of ¹²⁵I.

5. A matrix according to claim 4, wherein the decay has proceeded for from 2 to 150 hours.

6. A matrix according to any one of the claims 1-5, wherein the matrix is a metal.

7. A matrix according to claim 6, wherein the metal is titanium or copper.

8. A matrix according to any one of the claims 1-5, wherein the matrix is a non-metallic, biocompatible material.

9. A matrix according to any one of the claims 1-8, wherein the matrix is covered by a biocompatible material.

10. A matrix according to any one of the claims 1-9, comprising more than 0.001 % by weight of at least one element selected from the group consisting of iron, cobalt, zinc, manganese, platinum and iridium.

11. A medical device comprising a matrix according to any one of the claims 1-10.

12. The medical device of claim 11 that is a stent.

13. The medical device of claim 11 that is a radiotherapy seed.
